# EUROPEAN PATENT SPECIFICATION

(11) **EP 0 962 230 B1**
(45) Date of publication and mention of the grant of the patent: **30.06.2004**
(21) Application number: 99118538.0
(22) Date of filing: 07.11.1990
(51) Int. Cl.: A61M 5/32, A61M 5/50, A61M 5/31

(54) **Needle device, particularly hypodermic needle**
Nadelvorrichtung, insbesondere Subkutannadel
Dispositif d'aiguille, en particulier aiguille hypodermique

(43) Date of publication of application: 08.12.1999
(62) Divisional of application: 96114810.3
(73) Proprietor: MDC Investment Holdings, Inc., Wilmington, Delaware (US)
(72) Inventor: Botich, Michael J., Oxnard, CA 93035 (US); Halseth, Thor R., Simi Valley, CA 93065 (US)
(74) Representative: Newby, Martin John

(56) References cited:
- EP-A- 0 347 742
- WO-A-89/00435
- WO-A-90/07948
- CH-A- 669 910
- GB-A- 2 197 792
- US-A- 4 955 870
- US-A- 5 049 133

## Description

This invention relates generally to needle devices, e.g. hypodermic needles, and particularly to needle devices that are particularly suited for quickly and effectively removing the sharp injection needle which poses a serious health threat.

Various types of hypodermic needles currently exist in the art, with the object being to provide a protective cover or cap over the possibly wound-inflicting needle. Needles found in hypodermic syringes must be very sharp to quickly and easily puncture the skin of the patient in order to provide medicinals beneath the layer of skin. Additionally, the hypodermic needle is usually very thin and hard to see, especially in low-light conditions. Oftentimes, doctors and nurses accidentally prick themselves with the needle, either prior to or after an injection of a patient.

Pricking oneself prior to the injection of a solution does not present much of a health risk since the needles to be used are usually sterilized. Also, hypodermic syringes usually come with a needle cap which is secured over the top of the needle to prevent the accidental puncturing of skin. When the doctor or nurse takes off the needle cap, exposing the needle, there is usually little risk of being injured by the needle. However, upon placing the needle cap back onto the needle, oftentimes the fingers can be pricked by a slight visual miscalculation or by a motorneuro mistake. The consequences of this type of accident are more extreme.

Since the needle has already punctured the skin of the patient, blood and body fluid along with the viruses or bacteria which may be found in the patient could possibly be transferred to the health care provider by a single accidental prick. Various types of diseases previously known could be conveyed by such an accident, including hepatitis and cholera.

In the last decade, an even more menacing and lethal virus, the Acquired Immunity Deficiency Syndrome, or AIDS virus, is easily communicated by such an accidental and catastrophic event. Since there is no known cure for AIDS at this time, a great deal of care is required to prevent the accidental prick of the health care provider by a hypodermic needle which has previously been used on a patient.

Many types of syringes have been developed in an effort to address this problem, yet allow the ease of use of more conventional hypodermic needles.

Many of those devices are herein described below:
US-A-3,134,380 issued to T. Armao on May 26, 1964 discloses a hypodermic syringe needle having a shield which need not be removed prior to the use of the needle and which can be disposed of along with the needle itself. Holes are provided near the end of the shield to permit the escape of air as the shield is collapsed allowing the needle to protrude through the protective caps. The cap is held in an extended position by a spring which yields upon injection.
US-A-3,890,971 issued to T. A. Leeson on June 24, 1975 discloses a safety syringe for one time use including a plunger which is lockable by detent members and a slidable needle cap which is also permanently lockable to prevent reuse. The needle cap slides over the exterior of the syringe barrel and over the fixed needle.
US-A-4,367,738 issued to R. Legendre on January 11, 1983 discloses a pre-filled syringe having spikes upon the plunger rods to prevent the withdrawal of the plunger from the syringe barrel. No means is disclosed for protecting the tip of the needle from accidental pricking.
US-A-4,416,663 issued to R. N. Hall on November 22, 1983 discloses a self sterilizing needle, wherein a capsule containing sterilizing fluid and having perforated ends of flexible material with elastic memory tendencies for self sealing after actual penetration by the forward end of the needle. The capsule is coaxially and slidably received over the forward end of the needle with the forward exposed end of the needle slidably penetrating one end of the capsule and perforation for sterilizing of the needle. A syringe is provided for axially urging and positioning the capsule outward to its original position of rest. Then, the exposed end of the needle is again enclosed in the capsule for sterilization when the hypodermic penetration force is removed.
US-A-4,631,057 issued to C. B. Mitchell on December 23, 1986 discloses a needle coupled to a syringe barrel, wherein a needle guard is mounted on the barrel for movement from a retracted position in which the guard does not shield the needle to an extended position in which the guard shields the needle.
US-A-4,695,274 issued to R. L. Fox on September 22, 1987 discloses a safety needle attachment wherein the needle is initially and entirely surrounded by a protecting jacket which is releasably interlocked with a holder. When the needle is to be used, an interlocker is released and the jacket is effectively telescoped over the holder to project the needle through a membrane over the end of the jacket to a working position.
US-A-4,702,739 issued to N. M. Milorad on October 27, 1987 discloses a hypodermic needle having a sleeve extending from a holder protectively covering the needle so that the sleeve can be placed against the body part where injection is to occur and with the needle tip end proximate the body part. By sliding the holder toward the body part a detent restraint holding the sleeve in an extended position is overcome and relative retraction movement effected therewith.
US-A-4,731,068 issued to J. E. Hesse on March 15, 1988 discloses a non-reloadable syringe wherein the plunger is permitted to be withdrawn for purposes of loading the syringe and permitted to be urged forward to discharge the contents of the syringe. However, means is provided wherein subsequent retraction of the plunger assembly is inhibited to prevent further loading and use of the syringe.
US-A-4,735,618 issued to J. Hagen on April 5, 1988 discloses a protective enclosure for a hypodermic syringe needle formed by a tubular sleeve sized for friction fitting engagement over the barrel portion of the syringe. A needle guard portion is located at an opposed end and pivotally removable arms operate to permit the needle to pass through a central channel of the needle guard.
US-A-4,737,144 issued to P. V. Choksi issued April 12, 1988 discloses a syringe system comprising a tubular barrel and a sleeve mounted on the barrel to slide lengthwise from a retracted position in which the needle is exposed, and an extended position in which the sleeve extends protectively about the needle.
US-A-4,737,150 issued to H. Baeumle on April 12, 1988 discloses a two-cannula syringe, the first cannula being disposed so as to be displaced relative to the second cannula to be removable or displaceable in the longitudinal direction of the syringe.
US-A-4,738,663 issued to David E. Bogan on April 19, 1988 discloses a sleeve guide having a pair of fasteners with cavities formed in them that fit over the flange of the type which are located on hypodermic syringes for grasping in the user's fingers. The guide in the retracted position prevents accidental pricking by the needle.
US-A-4,743,233 issued to Michael B. Schneider on May 10, 1988 discloses a slidable sleeve over a syringe barrel that is connectable in a first position extending over a hypodermic needle and that is reconnectable in a second position along the syringe barrel to expose the needle for use.
US-A-4,747,829 issued to J. Jacob et al on May 31, 1988 discloses a pre-filled syringe with a retractable needle. A barrel of the syringe is removable within a casing from a remote pre-injection position to a forward injection position and back again. The barrel is moved forward allowing the needle to pass through an opening in a cap prior to injection.
US-A-4,747,830 issued to W. W. Gloyer et al on May 31, 1988 discloses a syringe having, a hollow barrel formed at the distal end to receive an injection piston carried by the plunger member which allows the needle to also to retract within the barrel by extracting the piston.
US-A-4,752,290 issued to J. J. Schramm on June 21, 1988 discloses a tubular shield which is adapted to protect users from injury. The tubular shield cooperates with the raised surfaces on the body of the medical appliance to be protected.
US-A-4,755,170 issued to T. A. Golden on July 5, 1988 discloses a protective sealing device comprising a block with which a sharp end of the needle can be held within to prevent accidental puncture. Also disclosed is a retaining shield which can be retracted over the needle to prevent accidental puncture.
US-A-4,772,272 issued to B. C. McFarland on September 20, 1988 discloses a protective sleeve for a hypodermic needle which sleeve is completely dissociable from the hypodermic syringe. The protective sleeve is moved over the needle protecting position to the needle injection position solely by axially movement of the protective sleeve.

CH-A-669 910 discloses a needle device having a separate intermediate cylinder into which a retracted needle is received. A hollow plunger is provided but it does not have a severable forward end.

It is desirable that the hypodermic needle can be made available in a safe condition prior to injection so that the health care provider will not accidentally prick his finger and require a new sterilized needle prior to the injection of the patient. It is also a requirement that after injection using the hypodermic needle, the needle can be safely and easily discarded without representing a continued health risk to anyone who may encounter the hypodermic needle, either on the premises of the health care facility, or in transit or arrival at the refuse collection area or dump.

There is potentially a great interest in the health care industry to manufacture, sell, distribute and use a hypodermic needle that provides the type of safety as described above. It can be easily operated using one hand, proves to be completely reliable and is easily and cheaply manufactured, yet still has a great deal of versatility for various applications using various needles in diameter and length.

The features described above as being desirable above for needle devices are all provided for by the present invention.

According to the present invention there is provided a needle device as claimed in the ensuing claim 1.

The present invention is embodied in an approved needle device which may be made entirely safe prior to injection due to a protective cover tip. Furthermore, after injection, the needle device is entirely safe, since a health care provider, using one hand, can retract the needle assembly into an isolation container which can be easily and safely discarded, preventing the injury or transmission of any dangerous viruses or bacteria. In addition, the needle device is easily manufactured, easy to use, and is able to provide visual and audible confirmation that the needle assembly has been safely retracted after injection.

More particularly, the needle device comprises a retractable needle assembly which can be safely, quickly, and easily retracted within a specially designed plunger. Furthermore, the plunger is fixedly held within a specially designed barrel. The barrel, plunger, and needle assembly can be easily discarded without the dangers associated with an exposed needle or needle that can be easily uncapped.

In a more specific embodiment of the invention, the needle assembly has a sharp end, a shaft with an axial passageway therethrough, and a holder defining a raised lip. The needle assembly includes a needle retainer having axially extending resilient fingers which are spreadable radially outwardly. The housing may also have tabs extending radially outwardly on an external surface of the housing to provide a mechanism for associating the housing with the barrel. The housing includes a first sealing means for providing a seal between the housing and the holder of the needle device. Also, there can be provided a second sealing means for providing a seal between the housing and the barrel.

An opening in a second end of the housing is suitably sized to receive the shaft of the needle assembly while retaining the holder when both are forwardly positioned within the housing. The resilient fingers of the housing have radially inwardly positioned hooks sized to engage and hold the raised lip of the holder of the needle assembly when the shaft of the needle assembly is forwardly located within the housing. The hooks have inwardly tapered shoulders so as to be easily spread by complementing and outwardly positioned tapered shoulders of the plunger.

The biasing means conveniently comprises a coiled spring positioned axially within the housing between the holder and the second end of the housing. The spring exerts an expansive force between the holder of the needle assembly and the second end of the housing, a force which is less than the retaining force exerted by the hooks of the resilient fingers, thereby retaining the needle assembly within the housing against the expansive force of the spring.

The barrel includes slots and grooves sized and positioned to receive the extending tabs of the housing and the second sealing means to provide an air-tight seal between the exterior of the housing and an interior of the barrel. The interior of the barrel is shaped sufficient to engage the housing and allow the resilient legs to flex radially outwardly.

The plunger is sized to be received concentrically within the barrel and has a hollow, axially located chamber or interior cavity therein. The plunger has a severable forward end with the outwardly tapered shoulders adjacent to said chamber. The severable forward end of the cavity disassociates from the plunger and is injected into the chamber when the outward tapered shoulders forcibly engage the inwardly tapered shoulders of the hooks of the resilient fingers, thereby spreading the resilient fingers outwardly and disengaging the hooks from the raised lip of the holder of needle assembly. The severable forward end dissociates under a pre-determined normal force between the holder of the needle assembly and the severable forward end. This allows the coiled spring to eject the needle assembly from the housing into the chamber within the plunger.

The chamber located within the plunger may be evacuated so that when the frangible end breaks and ejects with the needle assembly into the chamber, any peripheral fluid is also drawn into the chamber and away from the housing.

Furthermore, an inwardly-oriented lip on the exterior of the plunger is positioned so as to engage the complementing slot within the interior of the barrel when the plunger is in a fully-depressed position to lock the plunger within the barrel, preventing its removal and access to the retained needle assembly therein.

The holder of the needle device can have a bright colour (such as red) so as to be visually distinctive within the transparent syringe barrel and plunger, so that the health care provider can readily determine that the needle device is in a safe condition for transport or discard. The exterior of the barrel can also be fitted with a colour-coded sizing ring, which quickly and clearly identifies the size or capacity of the needle device.

The needle device of the present invention provides for a retractable needle assembly which is durable, disposable, easy to manufacture, prevents the accidental pricking after use, and provides for greater ease of handling the needle device after use, including the subsequent discarding of the device. The needle device is extremely simple in construction, yet completing effective in injecting fluid below the skin, subsequently becoming completely safe after the injection of the fluid while the health care provider need only use one hand to retract the needle assembly, leaving his other hand free. Furthermore, his or her fingers may remain in their relative positions to retract the needle assembly.

Other aspects and advantages of the present invention will be apparent from the following description of the preferred embodiments, taken in conjunction with the accompanying drawings, which illustrate, by way of example, the principals of the invention.
FIG. 1 is an exploded view of a needle device of the present invention;
FIG. 2 is a partial cross-sectional view of the needle device of the present invention, shown with its plunger proximate to the needle housing;
FIG. 3 is a cross-sectional view of the needle device of the present invention, with the needle housing, needle, and needle cap shown exploded from the barrel;
FIG. 4 is a cross-sectional view of the needle device in the present invention shown with the plunger in a partially depressed position within the barrel;
FIG. 5 is a cross-sectional view of the needle device of the present invention shown with the plunger in a fully depressed position and the needle fully retracted; and
FIG. 6 is a cross-sectional view of an alternative embodiment of a needle device of the present invention.

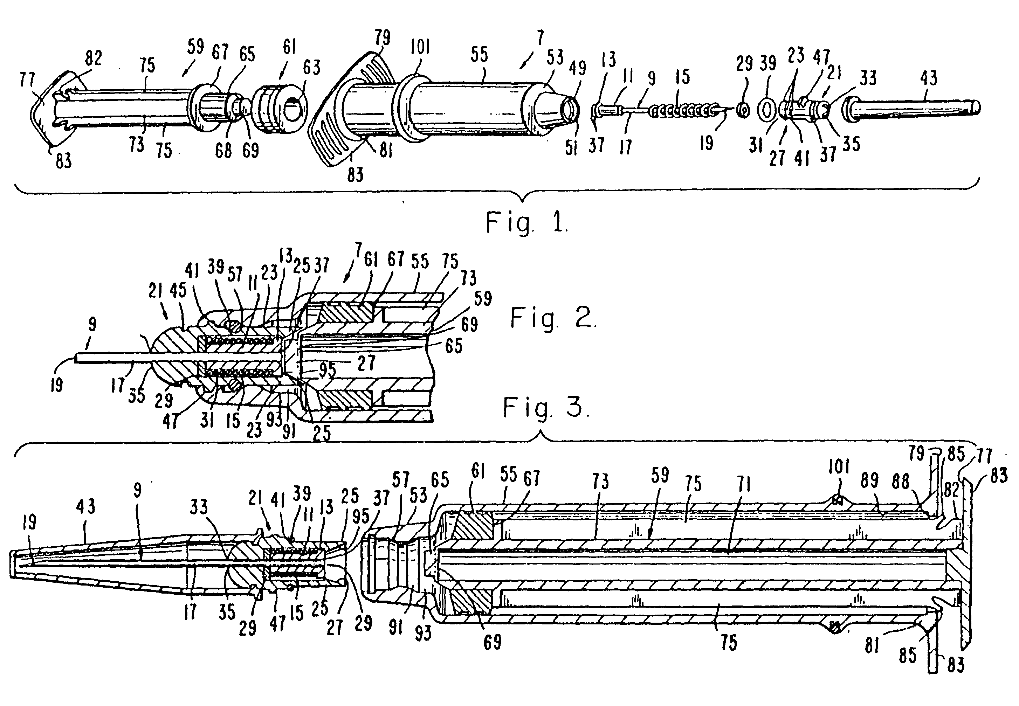

As shown in the drawings FIGS. 1-6, wherein like numerals represent like elements throughout, the needle device according to the invention is in the form of a hypodermic syringe 7 which is best shown in exploded view in FIG. 1.

The main components of the syringe 7 are a standard injection needle 9 having a specially mounted needle retainer or holder 11 including an enlarged lip 13, located posteriorly thereto. A coiled spring 15 rides a shaft 17 of the injection needle 9 with an axially located passageway 19 therethrough. A cylindrical spring housing 21 includes a plurality of radially spaced resilient fingers 23 which include inwardly engaging, and interiorally positioned, hooks 25 on the posterior end 27 of the spring housing 21. A sealing means or washer 29 is sized to be received within an inner cavity 31 of the spring housing 21.

The injection needle 9, including the enlarged lip 13 of the holder 11, can be forwardly positioned within the inner cavity 31 of the cylindrical spring housing 21. A cross-shaped opening 33 in a forward end 35 of the spring housing 21 allows the shaft 17 of the injection needle 9 to extend through the cross-shaped opening 33. The enlarged lip 13 is engaged by the hooks 25 when forwardly positioned within the spring housing 21, causing the resilient fingers 23 and hooks 25 to flex around the enlarged lip 13 and engage a top surface 37 of the enlarged lip 13.

The washer 29 provides a secure seal between the shaft 17 of the injection needle 9 and the inner cavity 31 of the spring housing 21. Finally, a gasket or O-ring 39 engages a circumferential groove 41, located midway between the posterior end 27 of the spring housing 21 and the forward end 35 of the spring housing 21. This configuration can be more clearly shown in FIG. 2, and also in FIG. 3, partially exploded from the other components of the hypodermic syringe 7.

Also, shown in FIG. 3 is a needle cap 43, which engages a forwardly-positioned second circumferential groove 45 of the spring housing 21. The spring housing 21 has radially extending bayonet tabs 47, which provide locking engagement within bayonet slots 49 and a bayonet groove 51, located within a tapered nose 53 of a syringe barrel 55. Engagement between the spring housing 21 and the tapered nose 53 of the syringe barrel 55 is easily accomplished by aligning the bayonet tabs 47 with the bayonet slots 49 and pushing the spring housing 21 through the bayonet slots 49 and then rotating the bayonet tabs 47 within the bayonet groove 51 to provide locking engagement therebetween. The bayonet tabs 47 may have slanted edges (not shown) on opposing sides and the bayonet groove may have raised surfaces (not shown) to allow the slanted edges to pass by the raised, surface in one direction of rotation. This effectively locks the spring housing 21 to the tapered nose 53 of the syringe barrel 55 in a ratchet-like manner.

The first tapered inner wall 57 within the tapered nose 53 of the syringe barrel 55 provides sealing engagement between the spring housing 21 and the syringe barrel 55, due to the tight fit of the O-ring 39 between the spring housing 21 and the first tapered inner wall 57.

A plunger 59 is sized to be received within the syringe barrel 55 and engages a plunger piston 61 of a conventional type commonly used with syringe systems known in the art, except that a cylindrical cavity 71 extends therethrough, allowing a frangible end or severable forward end 65 to enter the cylindrical cavity 71 of the plunger piston 61. The plunger piston 61 is positioned over the associated frangible end 65 and is supported by a rim 67. The length of the plunger piston 61 is such that outwardly tapered shoulders 68 extend through and above the passageway 63 of the plunger piston 61, joining the frangible end 65. Between the outwardly tapered shoulders 68 and the frangible end 65 is a circumferential groove 69 of a defined thickness of approximately 0.8 mm (1/32 of an inch), which allows the frangible end 65 to dissociate from the outwardly tapered shoulders 68 upon a normal force on the frangible end 65 of approximately 907 g (two pounds or less) in the preferred embodiment. The circumferential groove 69 can, of course, simply be a thinner construction of material allowing frangibility.

The plunger 59 includes the cylindrical central cavity 71 running axially through the plunger 59 and adjacent to the frangible end 65. The cylindrical cavity 71 has a diameter sufficient to allow the enlarged lip 13 and the holder 11 and the associated shaft 17 of the injection needle 9 to be injected into the cylindrical cavity 71 and need not be circular. Furthermore, the cylindrical cavity 71 can be evacuated so as to allow the vacuuming effect upon the dissociation of the frangible end 65 from the outwardly tapered shoulder 68.

A plunger sleeve 73 defines the cylindrical cavity 71 while reinforcement ribs 75 provide support to the plunger sleeve 73 and are associated with the rim 67 to provide additional support when the plunger 59 is being depressed. A pushing plate 77 is located on a posterior end 82 of the plunger 59. The pushing plate 77 is sized sufficiently to allow the thumb of a normal person to properly depress the plunger 59 when associated with the syringe barrel 55.

Also, finger retaining lips 79 are associated with the posterior end 81 of the syringe barrel 55 so as to allow the index finger and middle finger to grasp the finger-retaining lips 79 of the syringe barrel 55 while the thumb presses upon the pushing plate 77. Grooves 83 or knurling may be etched within the finger-retaining lips 79 or upon the pushing plate 77 to provide a greater coefficient of friction between the fingers and thumb and the finger retaining lip 79 and pushing plate 77, respectively.

Radially extending ratchet teeth 85 interrupt the reinforcing ribs 75 and are posteriorly located while being posteriorly flared to allow the ratchet teeth 85 to pass by an extending ratchet lip 88 defined by an interior wall 89 of the syringe barrel 55. Upon full depression of the syringe plunger 59 within the syringe barrel 55, the ratchet teeth 85 flex as they pass by the ratchet lip 88 and prevent the extraction of the plunger 59 from the syringe barrel 55.

In operation, the syringe 7, functions very much like a conventionally known hypodermic syringe as found in the prior art. However, after injection of the substance to be injected, the hyppodermic syringe 7 allows the dissociation of the severable forward end 65 from the outwardly tapered shoulders 68 of the plunger 59 and the radial flexing of the resilient fingers 23 so that the hooks 25 release the enlarged lip 13 of the holder 11 of the injection needle 9.

Since a circumferential space 91 exists between the resilient fingers 23, and the inner wall 93 of the syringe barrel 55, the resilient fingers 23 can flex, releasing the holder 11. The resilient fingers will only flex when inwardly tapered surfaces 95 of the hooks 25 are engaged by the outwardly tapered shoulders 68 of the plunger 59. Such engagement takes place when the plunger 59 is pushed through the syringe barrel 55 and the frangible end 65 abuts against the top surface 37 of the holder 11. A normal force of less than 907 g (2 pounds) exerted between the top surface 37 of the holder 11 and the frangible end 65 causes the frangible end 65 to dissociate from the outwardly tapered shoulders 68 of the plunger 59.

With the resilient fingers 23 flexed radially outwardly, causing the hooks 25 to release the holder 11, the compressed spring 15 exerts an ejecting force against the enlarged lip 13 of the holder 11, propelling the injection needle 9 along with the holder 11, as well as the dissociated frangible end 65 into the cylindrical cavity 71 of the plunger 59. The above operation makes a very distinctive click sound alerting the health care provider that the device is now safe.

Also, if the cylindrical cavity 71 is evacuated, a suction pulls any residual fluids into the cylindrical cavity 71. Upon further depression of the syringe plunger 59 into the syringe barrel 55, the ratchet teeth 85 engage the ratchet lip 88, preventing the plunger 59 from being extracted from the syringe barrel 55.

The holder 11 can be a bright red or fluorescent colour, while the plunger 59 and syringe barrel 55 can be manufactured from a transparent or translucent material so that the retracted position is readily identified in low light conditions and the needle is visibly safe for further handling, transport or discard.

Also, an interchangeable identification ring 101 can be positioned around the syringe barrel 55 so as to identify the hypodermic syringe 7 for whatever purpose.

The plunger 59, syringe barrel 55, holder 11, spring housing 21, and needle cap 43 can be made from a transparent or translucent plastics material. However, the spring housing 21 does not necessarily have to be transparent nor does the holder 11. Such materials and their manufacture are well known in the art and will not be further herein described. The plunger piston 61 can be formed of a neoprene material sufficient to provide a seal between the plunger piston 61 and the syringe barrel 55 and is also commonly known in the art and will not be hereinafter described in more detail. The shaft 17 of the injection needle 9 is of material known in the art as well.

The O-ring 39 can be of an elastomeric material, just as the washer 29 may also be of a resilient material, so as to provide a proper sealing effect well known in the art. It should be noted that the spring housing 21 must be formed of a durable plastics material which is resilient, so that the resilient fingers 23 properly and radially outwardly extend in association with the syringe plunger 59. The syringe plunger must be of a more resilient or brittle material or have a proper thickness so as not to flex inwardly when the frangible end 65 dissociates from the plunger 59. It is important that the plunger 59 remains durable to cause the resilient fingers 23 to move radially outwardly when the inwardly tapered surfaces 95 of the hooks 25 engage the outwardly tapered shoulders 68 of the syringe plunger 59. Specific examples of types of plastics and thicknesses are not required, as these can be readily determined by those ordinarily skilled in the art of plastics manufacture.

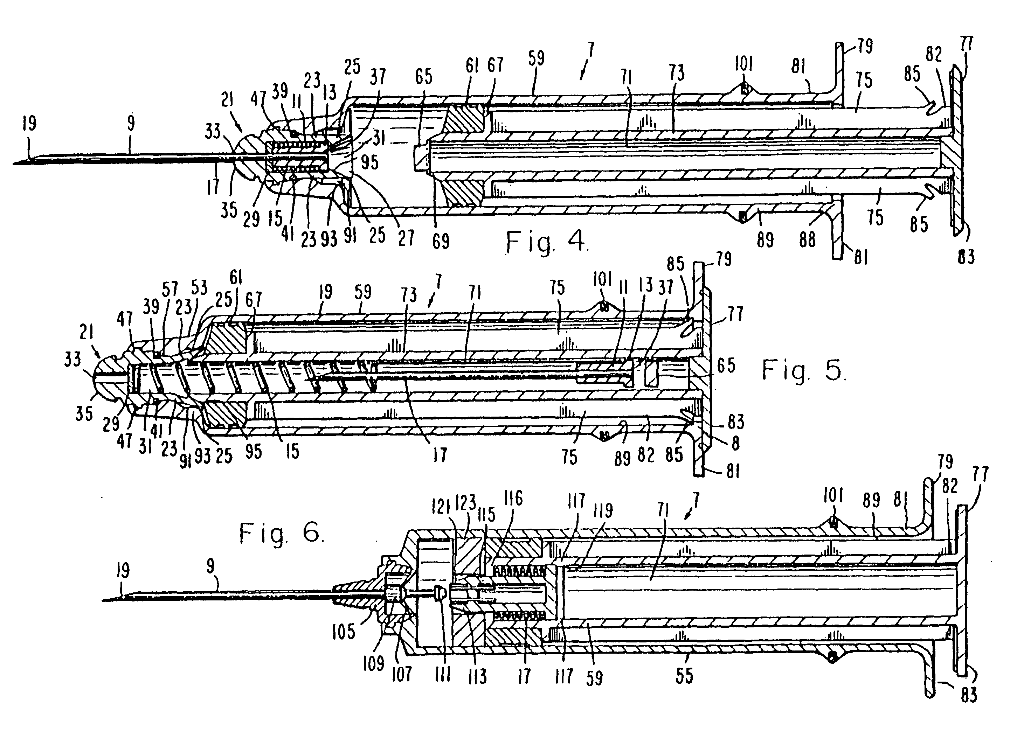

In an alternative embodiment, the mechanism responsible for ejecting the injection needle 9 can be fully positioned within the syringe plunger 59. As shown in FIG. 6, some slight variations in structure are necessary to achieve similar if not identical results as described in the first embodiment of the invention.

The injection needle 9 is held within a frangible needle holder 105, which includes a frangible cone 107, which engages an enlarged section 109 of the injection needle 9. The injection needle 9 has a length sufficient to extend well within the syringe barrel 59 and has an extraction end 111, which can be engaged by extraction hooks 113 of similar design as shown in FIGS. 1-5.

A needle retractor housing 115 is located and held on an inward end 117 of the syringe plunger 59, specifically held in place by detents 117, defined within the interior wall 119 of the cylindrical cavity 71 of the plunger 59. The compressed spring 17 exerts a force between the needle retracting housing 115 and the inner end 116 of the plunger 59. The force exerted by the spring is not sufficient to force the needle retractor housing 115 past the detents 117.

In operation the plunger 59 is pushed into the barrel 55 having outwardly tapered shoulders 121, which break the frangible cone 107, thereby releasing the enlarged section 109 of the injection needle 9. Further downward pressure on the plunger 59 forces the needle retractor housing 115 past detents 117, allowing the spring 15 to expand, pushing the needle retractor housing 115 deep within the cylindrical cavity 71 and taking with it the injection needle 9, because the hooks 113 grab the extraction end 111 as the needle retractor housing 115 is moved deeper into the cylindrical cavity 71 of the plunger 59. It should be noted that an extra piston spacer 123 is required for proper operation, due to the injection needle 9 extending within the syringe barrel 55.

Besides the above-identified differences, the second embodiment of the invention functions substantially as the first and the materials necessary for each of the components are similar to those materials as described in the first embodiment of the invention.

It should be appreciated from the foregoing description that the present invention describes an improved needle device with a retractable needle assembly which is simple in construction, yet completely effective in retracting a needle assembly once the needle assembly has served its purpose in the injection of fluids below the surface of the skin. The needle device of the present invention can be conveniently assembled from a minimum number of separate parts, all of which can be manufactured with relatively inexact precision, all of which are configured to facilitate compact and efficient operation. The needle device of the present invention can be fully and safely operated by the use of one hand to retract the needle assembly and allow for safe handling, transport, and discard.

Although the present invention has been described in detail with reference only to the presently-preferred embodiment, it will be appreciated by those of ordinary skill in the art that various modifications can be made without departing from the invention. Accordingly, the invention is limited only by the following claims.

## Claims

1. A needle device (7), comprising:
a hollow barrel (55) having a forward end and a first connector;
a needle assembly (9), comprising:
a needle having a sharpened tip operable between a projecting position in which the sharpened tip of the needle projects forwardly from the barrel (55), and a retracted position in which the sharpened tip is shielded against inadvertent contact;
a biasing element (15) biasing the needle toward the retracted position; and
a second connector that is cooperable with the first connector to attach the needle assembly to the barrel (55);
a needle retainer holding the needle in the projecting position against the bias of the biasing element (15); and
a plunger (59) slidably displaceable in the barrel (55) to a terminal position in which the plunger
is adjacent the forward end of the barrel, said plunger having an internal cavity (71), **characterized in that** said plunger (59) has a severable forward end (65) covering the forward end of the internal cavity (71),
wherein, upon pushing the rearward end of the plunger (59) forwardly after the plunger is in the terminal position, the severable forward end (65) of the plunger is severed and the needle is released from the projecting position so that the biasing element (15) displaces the needle into the retracted position in the said internal cavity (71).

2. A needle device (7) according to claim 1, further comprising a locking mechanism for locking the needle retainer to the barrel (55).

3. A needle device (7) according to claim 1 or claim 2, **characterised in that** said needle retainer further comprises a housing (21) for holding the needle assembly (9) and which includes axially extending resilient fingers (23) for retaining said needle in its projecting position in which the biasing element (15) exerts an expansive force between the housing (21) and needle assembly, and **in that** said plunger (59) has an engaging end which, on movement of the plunger forwardly of its terminal position, abuts against said resilient fingers (23) and spreads them resiliently outwardly to release the needle and enable the biasing element (15) to move the needle into its retracted position.

4. A needle device (7) according to claim 3, **characterised in that** said needle assembly (9) includes a holder (11) with a lip (13) which is retained by inwardly extending hook means (25) formed on the resilient fingers (23) when the needle is in its projecting position.

5. A needle device (7) according to claim 3 or 4, **characterised in that** said severable end (65) separates from the rest of the plunger (59) when a predetermined normal force is exerted between the severable end (65) and said needle assembly.

6. A needle device (7) according to claim 5, **characterised in that** said plunger (59) spreads said extending resilient fingers (23) radially outwards upon movement of the plunger (59) forwardly of its terminal position causing said severable end (65) to be broken and said biasing element (15) to propel said housing (21) and said needle into said interior cavity (71).

7. A needle device (7) according to any one of the preceding claims, **characterised in that** said needle retainer is operatively dissociable from a nose section (53) of said barrel (55).

8. A needle device (7) according to any one of the preceding of claims, **characterised by** a ratchet means (85, 88) for engaging said plunger (59) with said barrel (55), preventing separation of said plunger (59) from said barrel (55) after depression of the syringe plunger within the barrel to effect retraction of the needle.

9. A needle device (7) according to claim 7 or claim 8 when dependent on claim 7, **characterised in that** said needle retainer includes at least one radially extending tab (47) and **in that** said nose section (53) of the barrel includes at least one interior axial slot (49) extending to an inner radial slot (57), both sized to receive said tab(s) and allow secure engagement between said needle retainer when associated with said nose section (53) by positioning said tab(s) through said axial slot(s) and rotating said tab(s) within the said radial slot(s), thereby securing said needle retainer to said nose section.

10. A needle device (7) according to claim 4 or any one of claims 5 to 9 when dependent on claim 4, **characterised in that** said holder (11) has a highly visible colour and **in that** said plunger (59) and said barrel (55) are made of a translucent or transparent material to allow viewing of the holder (11), when the latter is ejected into the interior cavity (71) of said plunger, through said barrel (55).

11. A needle device (7) according to any one of the preceding claims, **characterised in that** said needle retainer has a head including an engagement groove (45) for engagement with the needle (43).

12. A needle device (7) according to claim 1, **characterised in that** said needle retainer comprises a housing (21) with resilient fingers (23) on one end which can be spread radially outwardly by contact therewith of the forward end of the plunger (55) to release the needle when the latter is in its projecting position captured in said housing, wherein said severable end (65) breaks allowing the captured needle to be propelled out of said housing and into said interior cavity (71) and to be retained therein, **in that** the plunger has tapered shoulders (68) adjacent said severable end (65) which engage opposite and complimentary shoulders (95) of said resilient fingers (23) allowing forward movement of the plunger (59) to spread the resilient fingers (23) radially outwards, and **in that** said needle device (7) further comprises an extending tab (35) and receiving slot means (49, 51) associated between the interior and exterior of the barrel (55), said tab (35) and receiving slot means (88, 89) being arranged to lock together when said plunger (59) is moved forwardly of said terminal position to lock the plunger (59) within the barrel (55).

13. A needle device (7) according to claim 12, **characterised in that** said barrel (55) and said plunger (59) are substantially transparent, said needle having a distinctive appearance to be readily seen through said plunger and said barrel once ejected into said interior cavity (71).

## Patentansprüche

1. Nadelvorrichtung (7) mit:
einem Hohlzylinder (55), der ein vorderes Ende und
einen ersten Verbinder aufweist;
einer Nadelanordnung (9), die Folgendes umfasst:
eine Nadel mit einer geschärften Spitze, die zwischen einer vorstehenden Position, in der die geschärfte Spitze der Nadel von dem Zylinder (55) nach vorne ragt, und einer zurückgezogenen Position, in der die geschärfte Spitze gegen unbeabsichtigten Kontakt geschützt ist, betätigt werden kann;
ein Vorbelastungselement (15), das die Nadel in die zurückgezogene Position vorbelastet; und
einen zweiten Verbinder, der mit dem ersten Verbinder zur Befestigung der Nadelanordnung am Zylinder (55) zusammenwirken kann;
einem Nadelhalter, der die Nadel gegen die Vorbelastung des Vorbelastungselements (15) in der vorstehenden Position hält; und
einem Stempel (59), der in dem Zylinder (55) gleitend in eine Endposition verschiebbar ist, in der sich der Stempel neben dem vorderen Ende des Zylinders befindet, wobei der Stempel einen Innenhohlraum (71) aufweist,
**dadurch gekennzeichnet, dass** der Stempel (59) ein abtrennbares vorderes Ende (65) aufweist, das das vordere Ende des Innenhohlraums (71) bedeckt,
wobei das abtrennbare vordere Ende (65) des Stempels bei Nachvorneschieben des hinteren Endes des Stempels (59), nachdem sich der Stempel in der Endposition befindet, abgetrennt wird und die Nadel aus der vorstehenden Position freigegeben wird, so dass das Vorbelastungselement (15) die Nadel in die zurückgezogene Position im Innenhohlraum (71) verschiebt.

2. Nadelvorrichtung (7) nach Anspruch 1, weiterhin mit einem Verriegelungsmechanismus zum Verriegeln des Nadelhalters am Zylinder (55).

3. Nadelvorrichtung (7) nach Anspruch 1 oder 2, **dadurch gekennzeichnet, dass** der Nadelhalter weiterhin ein Gehäuse (21) zum Halten der Nadelanordnung (9) umfasst, das sich axial erstreckende elastische Finger (23) zum Festhalten der Nadel in ihrer vorstehenden Position, in der das Vorbelastungselement (15) eine Ausdehnungskraft zwischen dem Gehäuse (21) und der Nadelanordnung ausübt, enthält, und dass der Stempel (59) ein Eingriffsende aufweist, das bei Bewegung des Stempels von seiner Endposition nach vorne an die elastischen Finger (23) stößt und sie elastisch nach außen spreizt, um die Nadel freizugeben und zu ermöglichen, dass das Vorbelastungselement (15) die Nadel in ihre zurückgezogene Position bewegt.

4. Nadelvorrichtung (7) nach Anspruch 3, **dadurch gekennzeichnet, dass** die Nadelanordnung (9) einen Halter (11) mit einer Lippe (13) enthält, die von einem sich nach innen erstreckenden, an den elastischen Fingern (23) ausgebildeten Hakenmittel (25) festgehalten wird, wenn sich die Nadel in ihrer vorstehenden Position befindet.

5. Nadelvorrichtung (7) nach Anspruch 3 oder 4, **dadurch gekennzeichnet, dass** sich das abtrennbare Ende (65) von dem Rest des Stempels (59) trennt, wenn eine vorbestimmte senkrechte Kraft zwischen dem abtrennbaren Ende (65) und der Nadelanordnung ausgeübt wird.

6. Nadelvorrichtung (7) nach Anspruch 5, **dadurch gekennzeichnet, dass** der Stempel die sich erstreckenden elastischen Finger (23) bei Nachvornebewegen des Stempels (59) von seiner Endposition radial nach außen spreizt, wodurch das abtrennbare Ende (65) zerbrochen wird und das Gehäuse (21) und die Nadel durch das Vorbelastungselement (15) in den Innenhohlraum (71) getrieben werden.

7. Nadelvorrichtung (7) nach einem der vorhergehenden Ansprüche, **dadurch gekennzeichnet, dass** der Nadelhalter von einem Nasenabschnitt (53) des Zylinders (55) operativ abtrennbar ist.

8. Nadelvorrichtung (7) nach einem der vorhergehenden Ansprüche, **gekennzeichnet durch** einen Sperrklinkenmechanismus (85, 88) zur Ineingriffnahme des Stempels (59) mit dem Zylinder (55), wodurch eine Trennung des Stempels (59) von dem Zylinder (55) nach Niederdrücken des Spritzenstempels in dem Zylinder zur Bewirkung eines Zurückziehens der Nadel verhindert wird.

9. Nadelvorrichtung (7) nach Anspruch 7 oder 8, sofern er von Anspruch 7 abhängig ist, **dadurch gekennzeichnet, dass** der Nadelhalter mindestens einen sich radial erstreckenden Lappen (47) enthält und dass der Nasenabschnitt (53) des Zylinders mindestens einen inneren, axialen Schlitz (49), der zu einem inneren, radialen Schlitz (57) verläuft, enthält, wobei beide zur Aufnahme des Lappens bzw. der Lappen bemessen sind und eine sichere Ineingriffnahme zwischen dem Nadelhalter bei Zuordnung zum Nasenabschnitt (53) gestatten, indem der bzw. die Lappen durch den bzw. die axialen Schlitz(e) positioniert und der bzw. die Lappen in dem bzw. den radialen Schlitz(en) gedreht wird bzw. werden, wodurch der Nadelhalter an dem Nasenabschnitt befestigt wird.

10. Nadelvorrichtung (7) nach Anspruch 4 oder einem der Ansprüche 5 bis 9, sofern er von Anspruch 4 abhängig ist, **dadurch gekennzeichnet, dass** der Halter (11) eine hochsichtbare Farbe aufweist und dass der Stempel (59) und der Zylinder (55) aus einem durchscheinenden oder durchsichtigen Material bestehen, so dass der Halter (11) durch den Zylinder (55) zu sehen ist, wenn Ersterer in den Innenhohlraum (71) des Stempels gestoßen wird.

11. Nadelvorrichtung (7) nach einem der vorhergehenden Ansprüche, **dadurch gekennzeichnet, dass** der Nadelhalter einen Kopf mit einer Eingriffsnut (45) zum Eingriff mit der Nadel (43) aufweist.

12. Nadelvorrichtung (7) nach Anspruch 1, **dadurch gekennzeichnet, dass** der Nadelhalter ein Gehäuse (21) mit elastischen Fingern (23) an einem Ende umfasst, die durch Kontakt des vorderen Endes des Stempels (55) damit radial nach außen gespreizt werden können, um die Nadel freizugeben, wenn sich Letztere in ihrer in dem Gehäuse festgelegten vorstehenden Position befindet, wobei das abtrennbare Ende (65) zerbricht und dadurch gestattet, dass die festgelegte Nadel aus dem Gehäuse heraus und in den Innenhohlraum (71) hinein getrieben und darin festgehalten wird, dass der Stempel sich verjüngende Schultern (68) neben dem abtrennbaren Ende (65) aufweist, die gegenüberliegende und komplementäre Schultern (95) der elastischen Finger (23) in Eingriff nehmen, wodurch sich der Stempel (59) zum radialen Nachaußenspreizen der elastischen Finger (23) vorwärts bewegen kann, und dass die Nadelvorrichtung weiterhin einen sich davon erstreckenden Lappen (35) und zwischen der Innenund Außenseite des Zylinders (55) zugeordnete Aufnahmeschlitzmittel (49, 51) umfasst, wobei der Lappen (35) und die Aufnahmeschlitzmittel (88, 89) so angeordnet sind, dass sie miteinander verriegelt werden, wenn der Stempel (59) von der Endposition nach vorne bewegt wird, um den Stempel (59) in dem Zylinder (55) zu verriegeln.

13. Nadelvorrichtung (7) nach Anspruch 12, **dadurch gekennzeichnet, dass** der Zylinder (55) und der Stempel (59) im Wesentlichen durchsichtig sind, wobei die Nadel auffällig aussieht, damit sie leicht durch den Stempel und den Zylinder zu sehen ist, nachdem sie in den Innenhohlraum (71) gestoßen worden ist.

## Revendications

1. Dispositif d'aiguille (7) comprenant :
un corps creux (55) ayant une extrémité avant et un premier connecteur ;
un ensemble d'aiguille (9) comprenant :
une aiguille ayant une pointe acérée capable de fonctionner entre une position en saillie dans laquelle la pointe acérée de l'aiguille fait saillie vers l'avant depuis le corps (55), et une position rétractée dans laquelle la pointe acérée est protégée contre tout contact accidentel ;
un élément de poussée (15) poussant l'aiguille vers la position rétractée ; et
un deuxième connecteur qui peut coopérer avec le premier connecteur pour attacher l'ensemble d'aiguille au corps (55) ;
un moyen de retenue d'aiguille retenant l'aiguille dans la position en saillie contre la poussée de l'élément de poussée (15) ; et
un plongeur (59) déplaçable à coulissement dans le corps (55) jusque dans une position terminale dans laquelle le plongeur est adjacent à l'extrémité avant du corps, ledit plongeur ayant une cavité intérieure (71), **caractérisé en ce que** ledit plongeur (59) a une extrémité avant cassable (65) recouvrant l'extrémité avant de la cavité intérieure (71),
où, sous l'effet de la poussée de l'extrémité arrière du plongeur (59) vers l'avant une fois que le plongeur est dans la position terminale, cette extrémité avant cassable (65) du plongeur se casse et l'aiguille est libérée de la position en saillie de sorte que l'élément de poussée (15) déplace l'aiguille dans la position rétractée dans ladite cavité intérieure (71).

2. Dispositif d'aiguille (7) selon la revendication 1, comprenant en outre un mécanisme de verrouillage pour verrouiller le moyen de retenue d'aiguille sur le corps (55).

3. Dispositif d'aiguille (7) selon la revendication 1 ou la revendication 2, **caractérisé en ce que** ledit moyen de retenue d'aiguille comprend en outre un logement (21) pour retenir l'ensemble d'aiguille (9) et qui comporte des doigts élastiques s'étendant axialement (23) pour retenir ladite aiguille dans sa position en saillie dans laquelle l'élément de poussée (15) exerce une force d'expansion entre le logement (21) et l'ensemble d'aiguille, et **en ce que** ledit plongeur (59) a une extrémité d'engagement qui, lors du mouvement du plongeur vers l'avant de sa position terminale, vient buter contre lesdits doigts élastiques (23) et les écarte élastiquement vers l'extérieur pour libérer l'aiguille et permettre à l'élément de poussée (15) de déplacer l'aiguille dans sa position rétractée.

4. Dispositif d'aiguille (7) selon la revendication 3, **caractérisé en ce que** ledit ensemble d'aiguille (9) comporte un support (11) avec une lèvre (13) qui est retenue par un moyen de crochet s'étendant vers l'intérieur (25) formé sur les doigts élastiques (23) lorsque l'aiguille est dans sa position en saillie.

5. Dispositif d'aiguille (7) selon la revendication 3 ou 4, **caractérisé en ce que** ladite extrémité cassable (65) se sépare du reste du plongeur (59) lorsqu'une force normale prédéterminée est exercée entre l'extrémité cassable (65) et ledit ensemble d'aiguille.

6. Dispositif d'aiguille (7) selon la revendication 5, **caractérisé en ce que** ledit plongeur (59) écarte lesdits doigts élastiques étendus (23) radialement vers l'extérieur lorsque le plongeur (59) se déplace vers l'avant de sa position terminale en provoquant la fracture de ladite extrémité cassable (65) et la propulsion par ledit élément de poussée (15) dudit logement (21) et de ladite aiguille dans ladite cavité intérieure (71).

7. Dispositif d'aiguille (7) selon l'une quelconque des revendications précédentes, **caractérisé en ce que** ledit moyen de retenue d'aiguille peut être dissocié fonctionnellement d'une section de nez (53) dudit corps (55).

8. Dispositif d'aiguille (7) selon l'une quelconque des revendications précédentes, **caractérisé par** un moyen d'encliquetage (85, 88) pour engager ledit plongeur (59) avec ledit corps (55), en empêchant la séparation dudit plongeur (59) dudit corps (55) après que le plongeur de seringue a été enfoncé dans le corps pour effectuer le retrait de l'aiguille.

9. Dispositif d'aiguille (7) selon la revendication 7 ou la revendication 8 lorsqu'elle dépend de la revendication 7, **caractérisé en ce que** ledit moyen de retenue d'aiguille comporte au moins une languette s'étendant radialement (47) et **en ce que** ladite section de nez (53) du corps comporte au moins une fente axiale intérieure (49) s'étendant vers une fente radiale interne (57), toutes deux étant dimensionnées de manière à recevoir la ou lesdites languettes et à permettre l'engagement sûr entre ledit moyen de retenue d'aiguille lorsqu'il est associé à ladite section de nez (53) en positionnant la ou lesdites languettes à travers la ou lesdites fentes axiales et en faisant tourner la ou lesdites languettes dans ladite fente radiale, en fixant ainsi ledit moyen de retenue d'aiguille sur ladite section de nez.

10. Dispositif d'aiguille (7) selon la revendication 4 ou l'une quelconque des revendications 5 à 9 lorsqu'elles dépendent de la revendication 4, **caractérisé en ce que** ledit support (11) a une couleur bien visible et **en ce que** ledit plongeur (59) et ledit corps (55) sont fabriqués en un matériau translucide ou transparent pour permettre de voir le support (11), lorsque ce dernier est éjecté dans la cavité intérieure (71) dudit plongeur, à travers ledit corps (55).

11. Dispositif d'aiguille (7) selon l'une quelconque des revendications précédentes, **caractérisé en ce que** ledit moyen de retenue d'aiguille possède une tête comportant une rainure d'engagement (45) pour l'engagement avec l'aiguille (43).

12. Dispositif d'aiguille (7) selon la revendication 1, **caractérisé en ce que** ledit moyen de retenue d'aiguille comprend un logement (21) avec des doigts élastiques (23) sur une extrémité, lesquels peuvent être écartés radialement vers l'extérieur par contact avec eux de l'extrémité avant du plongeur (55) pour libérer l'aiguille (9) lorsque cette dernière est dans sa position en saillie reçue dans ledit logement, ladite extrémité cassable (65) se cassant en permettant ainsi à l'aiguille reçue d'être projetée hors dudit logement et dans ladite cavité intérieure (71) et d'y être retenue, **en ce que** le plongeur a des épaulements effilés (68) adjacents à ladite extrémité cassable (65), qui engagent des épaulements opposés et complémentaires (95) desdits doigts élastiques (23) en permettant le mouvement vers l'avant du plongeur (59) pour écarter les doigts élastiques (23) radialement vers l'extérieur, et **en ce que** ledit dispositif d'aiguille (7) comprend en outre une languette étendue (35) et un moyen de fente de réception (49, 51) associé entre l'intérieur et l'extérieur du corps (55), ladite languette (35) et ledit moyen de fente de réception (88, 89) étant prévus pour se verrouiller ensemble lorsque ledit plongeur (59) est déplacé vers l'avant de ladite position terminale pour verrouiller le plongeur (59) dans le corps (55).

13. Dispositif d'aiguille (7) selon la revendication 12, **caractérisé en ce que** ledit corps (55) et ledit plongeur (59) sont substantiellement transparents, ladite aiguille ayant un aspect distinctif devant être facilement visible à travers ledit plongeur et ledit corps une fois éjectée dans ladite cavité intérieure (71).
